# EUROPEAN PATENT SPECIFICATION

(11) **EP 2 925 336 B1**
(45) Date of publication and mention of the grant of the patent: **12.10.2022**
(21) Application number: 13798341.7
(22) Date of filing: 29.11.2013
(51) Int. Cl.: A61K 36/76, A61K 31/34, A61K 36/15, A61P 11/06, A61P 11/08

(54) **NATURAL COMPOUNDS FOR USE IN THE TREATMENT OF BETA-2 ADRENERGIC RECEPTOR RELATED DISEASES**
NATÜRLICHE VERBINDUNGEN ZUR VERWENDUNG BEI DER BEHANDLUNG VON MIT DEM BETA-2-ADRENOREZEPTOR ASSOZIIERTEN ERKRANKUNGEN
COMPOSÉS NATURELS DESTINÉS À ÊTRE UTILISÉS DANS LE TRAITEMENT DES MALADIES LIÉES AUX RÉCEPTEURS BÊTA2 ADRÉNERGIQUES

(30) Priority: 30.11.2012 US 201261731582 P
(43) Date of publication of application: 07.10.2015
(73) Proprietor: Harmonic Pharma, 54500 Vandoeuvre les Nancy (FR); Norske Skog Golbey, 88194 Golbey Cedex (FR)
(72) Inventor: SOUCHET, Michel, F-91190 Gif-sur-Yvette (FR); KARABOGA, Arnaud Sinan, F-67100 Strasbourg (FR); GEGOUT, Stéphane, F-54000 Nancy (FR)
(74) Representative: Cabinet Becker et Associés
(86) International application number: PCT/EP2013/075134
(87) International publication number: WO 2014/083171

(56) References cited:
- EP-A1- 2 143 435
- WO-A1-03/045376
- WO-A1-03/061649
- US-A1- 2002 061 854
- US-A1- 2006 035 964
- US-B1- 6 486 126
- US-B1- 6 498 145
- BARANETCHI C ET AL: "Administration of fir hydralcoholic extract under aerosol form in respiratory tract infections", REVISTA MEDICO-CHIRURGICALA A SOCIETATII DE MEDICI SI NATURALISTI DIN LASI, SOCIETATEA DE MEDICI SI NATURALISTI, IASI, RO, vol. 89, no. 1, 1 January 1985 (1985-01-01), pages 123-124, XP009175203, ISSN: 0048-7848
- WESTCOTT NEIL D ET AL: "Flax seed lignan in disease prevention and health promotion", PHYTOCHEMISTRY REVIEWS, KLUWER, NL, vol. 2, no. 3, 1 January 2003 (2003-01-01) , pages 401-417, XP002407509, ISSN: 1568-7767, DOI: 10.1023/B:PHYT.0000046174.97809.B6
- DATABASE WPI Week 201135 Thomson Scientific, London, GB; AN 2011-E94477 XP002718102, & CN 101 991 566 A (UNIV PLA SECOND MILITARY MEDICAL) 30 March 2011 (2011-03-30)
- COSENTINO M ET AL: "Immunomodulatory activity of the lignan 7-hydroxymatairesinol potassium acetate (HMR/lignan@?) extracted from the heartwood of Norway spruce (Picea abies)", INTERNATIONAL IMMUNOPHARMACOLOGY, ELSEVIER, AMSTERDAM, NL, vol. 10, no. 3, 1 March 2010 (2010-03-01), pages 339-343, XP026919584, ISSN: 1567-5769, DOI: 10.1016/J.INTIMP.2009.12.005 [retrieved on 2009-12-11]
- BJARNE HOLMBOM ET AL: "Knots in trees A new rich source of lignans", PHYTOCHEMISTRY REVIEWS, KLUWER, NL, vol. 2, no. 3, 1 October 2003 (2003-10-01) , pages 331-340, XP008104241, ISSN: 1568-7767, DOI: 10.1023/B:PHYT.0000045493.95074.A8 [retrieved on 2004-11-08]
- BJARNE HOLMBOM ET AL: "Knots in trees ? A new rich source of lignans", PHYTOCHEMISTRY REVIEWS, KLUWER, NL, vol. 2, no. 3, 1 October 2003 (2003-10-01) , pages 331-340, XP008104241, ISSN: 1568-7767, DOI: 10.1023/B:PHYT.0000045493.95074.A8 [retrieved on 2004-11-08]

## Description

### FIELD OF INVENTION

The present disclosure relates to the treatment and prevention of β2-adrenergic receptor related diseases. More specifically, the present invention relates to the use of natural compounds that may be extracted from knot-wood for treating or preventing COPD.

### BACKGROUND OF INVENTION

Asthma and chronic obstructive pulmonary disease (COPD) are both prevalent and chronic respiratory diseases that affect millions of people worldwide. Although the etiology of asthma and COPD is not completely understood, smooth muscle dysfunction and chronic inflammation are known to play important roles in the pathophysiology of these diseases. Thus, both anti-inflammatory and bronchodilator medicines are used extensively in this context. The β2-adrenoceptor agonists (β2-agonists) are the most effective bronchodilators, offering proven benefits in reducing the burden of asthma and COPD.

Activation of the β2-adrenoceptor results in the activation of adenylyl cyclase (AC) via a stimulatory G-protein (Gs) and an increase in the cAMP concentration which is a key regulator of numerous signalling cascades.

Several novel β2-adrenoceptor (AR) agonists, new combination platforms such as dual-acting muscarinic antagonist-β2-AR agonist bronchodilators, xanthine drugs and phosphodiesterase inhibitors, and their combination with another bronchodilator class, or an inhaled corticosteroid are currently under development with the aim of achieving once-daily dosing.

For example, the international patent application WO 2012/098495 describes a pharmaceutical composition comprising a phosphodiesterase-4 (PDE-4) enzyme inhibitor (such as, for example, benzofuropyridine) and a β2 adrenergic receptor agonist, and the use of this pharmaceutical composition for treating a respiratory disorder, preferably asthma and/or COPD.

Moreover, the European patent application EP 2 386 555 describes new cyclohexylamide derivatives having both β2 adrenergic receptor agonist and m3 muscarinic receptor antagonist activities, and the use of these compounds for the treatment of respiratory disorders, such as, for example, asthma and COPD.

However, due to the fact that asthma and COPD are highly disabling diseases, there is still a need for new improved treatments, allowing for example decreasing the frequency of drug administration.

WO 03/061649 discloses the use of lignans extracted from heart wood of spruce (Picea abies) for the treatment of cancer and cardiovascular diseases. EP2143435 discloses knot-wood extracts comprising secoisolariciresinol for the treatment of cancer. US 6,486,126 discloses the use of compositions comprising secoisolariciresinol for the treatment of atherosclerosis, diabetes, ischemic heart disease, heart failure, endotoxic and hemorrhagic shock, inflammatory bowel disease, rheumatoid arthritis, Parkinson's disease, and stroke. US 6,498,145 discloses the use of compositions comprising secoisolariciresinol for the treatment of hypertension. Baranetchi et al. (Revista Medico-Chirurgicala a Societatii de Medici Si Naturalisti Din Lasi, Societatea de Medici si Naturalisti, Iasi, RO, vol. 89, 1985, 123-124) disclose the use of pine extracts for the treatment of asthma. Westcott et al. (Phytochemistry reviews, 2: 401-417, 2003) disclose the use of flax seed lignans for the treatment of atherosclerosis, cancer and diabetes. CN101991566 discloses the use of lariciresinol for the treatment of diabetes, immune diseases, cardiac diseases and asthma. US 2006/0035964 discloses the use of lignans in Hongdoushan plant as a hypoglycemic agent, a liver protecting agent, and an anticancer agent. WO 03/045376 discloses the use of hydroxymatairesinol for the treatment of rheumatoid arthritis, asthma, inflammatory bowel disease or an inflammatory condition of the skin, HIV, AIDS, psoriasis, Parkinson's disease, Alzheimer's disease, an autoimmune disease, diabetes, hypocholesterolemic atherosclerosis, cataract or amyotrophic lateral sclerosis. US 2002/0061854 discloses the use of *Picea abies* knot-wood extracts for treating cancer and cardiovascular diseases. Cosentino et al. (International immunopharmacology 2010, 10, 339-343) disclose the immunomodulatory activity of hydroxymatairesinol from *Picea abies.* Holmbom et al. (Phytochemistry Reviews 2: 331-340, 2003) disclose the general occurrence of lignans in coniferous tree knots.

The Inventors herein surprisingly show that a knot-wood extract, comprising lignans, exhibits an elevated β2 adrenergic receptor agonist activity, and may thus be a promising alternative to existing compounds currently used for treating COPD.

### SUMMARY

The subject-matter of the present invention is such as defined in claims 1-13.

The present disclosure thus relates to a knot-wood extract comprising at least one lignan selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof.

Another object of the disclosure is a method of activating a β2 adrenergic receptor, comprising administering an effective amount of a knot-wood extract as disclosed herein; or an effective amount of at least one lignan selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof.

The present disclosure also relates to a method of treating a β2 adrenergic receptor related disease in a subject in need thereof, comprising administering to the subject an effective amount of a knot-wood extract as disclosed herein, or an effective amount of at least one lignan selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof.

In one embodiment, the β2 adrenergic receptor related disease is selected from the list comprising pulmonary diseases; pre-term labor; neurological disorders; cardiac disorders; fibrosis; inflammation; atopic dermatitis; rheumatoid arthritis; thrombosis; allergies; ophthalmic diseases; urological disorders; gastrointestinal disorders; metabolic disorders and cancers. Preferably, the β2 adrenergic receptor related disease is chronic obstructive pulmonary disease (COPD).

The present disclosure also relates to the use of a knot-wood extract as disclosed herein, or of at least one lignan selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof, for activating a β2 adrenergic receptor.

The present disclosure also relates to the use of a knot-wood extract as disclosed herein, or at least one lignan selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof, for treating, or for use in treating, a β2 adrenergic receptor related disease in a subject in need thereof.

In one embodiment, said β2 adrenergic receptor related disease is selected from the list comprising pulmonary diseases; pre-term labor; neurological disorders; cardiac disorders; fibrosis; inflammation; atopic dermatitis; rheumatoid arthritis; thrombosis; allergies; ophthalmic diseases; urological disorders; gastrointestinal disorders; metabolic disorders and cancers. Preferably, said β2 adrenergic receptor related disease is chronic obstructive pulmonary disease (COPD).

The present disclosure also relates to a knot-wood extract as hereinabove described, or to at least one lignan selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof, for use in neuroprotection and/or cardioprotection to a subject in need thereof.

### DEFINITIONS

In the present invention, the following terms have the following meanings:
- **"β2-adrenergic receptor related disease or disorder":** includes all medical conditions alleviated by treatment with a beta-2 adrenergic receptor agonist and includes all disease states and/or conditions that are acknowledged now, or that will be found in the future, to be associated with beta-2 adrenergic receptor activity.
- **"Treatment"** refers to both therapeutic treatment and prophylactic or preventative measures; wherein the object is to prevent or slow down (lessen) the targeted β2 adrenergic receptor related disease or disorder. Those in need of treatment include those already with the disease or disorder as well as those prone to have the disease or disorder or those in whom the disease or disorder is to be prevented. A subject or mammal is successfully "treated" for a disease or disorder if, after receiving a therapeutic amount of an extract or a lignan for use according to the invention, the patient shows observable and/or measurable reduction in or absence of one or more of the following: reduction in the number of pathogenic cells; reduction in the percent of total cells that are pathogenic; and/or relief to some extent, one or more of the symptoms associated with the specific disease or disorder; reduced morbidity and mortality, and improvement in quality of life issues. The above parameters for assessing successful treatment and improvement in the disease or disorder are readily measurable by routine procedures familiar to a physician.

- **"Effective amount"** means level or amount of agent that is aimed at, without causing significant negative or adverse side effects to the target, (1) delaying or preventing the onset of a β2 adrenergic receptor related disease or disorder; (2) slowing down or stopping the progression, aggravation, or deterioration of one or more symptoms of the β2 adrenergic receptor related disease or disorder; (3) bringing about ameliorations of the symptoms of the β2 adrenergic receptor related disease or disorder; (4) reducing the severity or incidence of the β2 adrenergic receptor related disease or disorder; or (5) curing the β2 adrenergic receptor related disease or disorder. An effective amount may be administered prior to the onset of the β2 adrenergic receptor related disease or disorder, for a prophylactic or preventive action. Alternatively or additionally, the effective amount may be administered after initiation of the β2 adrenergic receptor related disease or disorder, for a therapeutic action.
- **"Pharmaceutically acceptable excipient"** refers to an excipient that does not produce an adverse, allergic or other untoward reaction when administered to an animal, preferably a human. It includes any and all solvents, dispersion media, coatings, antibacterial, antiparasitic, and antifungal agents, isotonic and absorption delaying agents and the like. For human administration, preparations should meet sterility, pyrogenicity, general safety and purity standards as required by FDA Office of Biologics standards.
- **"Lignan"** refers to a group of chemical compounds found in plants. Plant lignans are polyphenolic substances, derivatives, esters and salts thereof.
- **"Hydroxymatairesinol"** refers to a lignan having the following formula 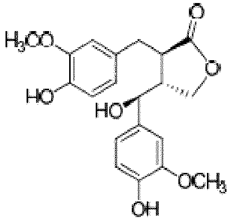
- **"Epimers"** refers to diastereoisomers that differ in configuration of only one stereogenic center, wherein diastereoisomers are a class of stereoisomers that are non-superimposable, non-mirror images of one another. In one embodiment, "epimers of hydroxymatairesinol" refers to the diastereoisomers 7S,8R,8'R and 7R,8R,8'R. 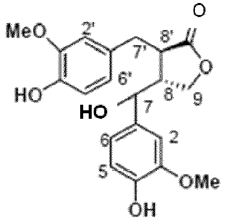
- **"Lariciresinol"** refers to a lignan having the following formula: 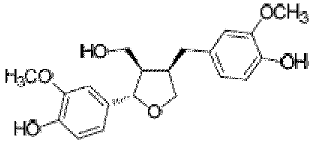
- **"Secoisolariciresinol"** refers to a lignan having the following formula: 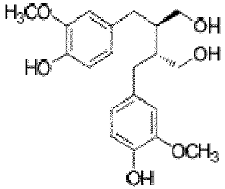
- **"Liovil"** refers to a lignan having the following formula:

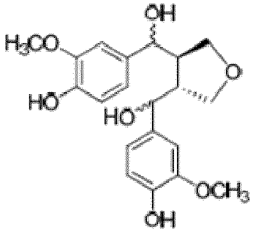

### DETAILED DESCRIPTION

The present disclosure thus relates to a lignan-containing knot-wood extract.

In one embodiment, the knot-wood extract exhibits a β2 adrenergic receptor agonist activity *in vitro.* Methods for measuring the β2 adrenergic receptor agonist activity *in vitro* are well-known from the skilled artisan. An example of such a method is disclosed in the Examples. Briefly, said method includes incubating recombinant mammalian cells expressing a human β2 adrenergic receptor, such as, for example, recombinant CHO cells, with the tested compounds for about 30 minutes at room temperature, and measuring the accumulation of cAMP, such as, for example, by homogeneous time resolved fluorescence (HTRF) technology.

In one embodiment, the knot-wood extract is a dry extract.

In one embodiment, the extraction is carried out in presence of a solvent selected from the group comprising water, alkanes, such as, for example, hexane; halogenated alkanes, such as, for example, chloroform or dichloromethane; ethers, such as, for example, diethyl ether; esters, such as, for example, ethyl acetate; alcohols, such as, for example, ethanol; ketones, such as, for example, acetone; aromatic solvents, especially toluene; or mixtures thereof, such as for example toluene/ethanol.

In one embodiment, the extraction process is a supercritical process.

Ine one embodiment, the extraction process is an enzymatic process.

In one embodiment, the extraction process is a soxhlet based extraction process, preferably using acetone as a solvent. An example of such a process is shown in Example 1.

The Inventors have shown that the knot-wood extract as hereinabove described comprises one or more lignans, especially selected from the list comprising or consisting of (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof.

In one embodiment, the knot-wood extract comprises at least 10% of lignans, preferably more than 15% of lignans. Preferably, the lignans are selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof, and mixtures thereof. In one embodiment, the content in lignans may be measured using a gas chromatography apparatus coupled with a mass spectrometer.

In one embodiment, the extract as disclosed herein comprises at least one lignan, preferably selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof at a concentration of at least 0.05% in weight to the total weight of the extract, preferably of at least 0.1% w/w, more preferably of at least 1% w/w, and even more preferably at a concentration ranging from 1 to 30%.

In one embodiment, the extract as disclosed herein consists essentially of lignans, preferably selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof.

Another object of the disclosure is a knot-wood fraction comprising at least one lignan, preferably selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof at a concentration of at least 10% in weight to the total weight of the fraction, preferably of at least 20% w/w.

In one embodiment, the knot-wood fraction is a dry fraction.

In one embodiment, the fraction is recovered by fractionation of a knot-wood extract as disclosed herein. Examples of fractionation methods include, but are not limited to precipitation, crystallization, normal-phase liquid chromatography on a solid support, reversed-phase liquid chromatography on a solid support, atmospheric pressure liquid chromatography, medium pressure liquid chromatography, high performance liquid chromatography, flash-chromatography, countercurrent chromatography, or by any combination of these methods.

In one embodiment of the invention, the knot-wood extract or fraction is an extract or a fraction from at least one resinous tree, preferably from *Abies alba, Picea abies, Betula pendula, Pinus sylvestris, Abies sibirica, Pinus sibirica, Abies balsamea, Thuja occidentalis, Northern white-cedar, Pinus cembra, Pseudotsuga menziesii (douglas), Larix decidua, Picea glauca, Picea mariana, Picea pingens, Abies pectinata, Abies lasiocarpa, Pinus banksiana, Pinus resinosa, Larix lariciana, Larix sibirica, Thuja plicata, Fagus Sylvatica, Populus tremula, Populus tremulus, Tsuga heterophylla, Pinus contorta,* and *Juniperus virginiana (red cedar).*

Preferably, the knot-wood extract is an extract from *Abies alba* and/or *Picea abies.*

The present disclosure also relates to at least one lignan, preferably selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof.

In one embodiment, the at least one lignan as disclosed herein exhibits a β2 adrenergic receptor agonist activity *in vitro.* Methods for measuring the β2 adrenergic receptor agonist activity *in vitro* are well-known from the skilled artisan. An example of such a method is disclosed in the Examples. Briefly, said method includes incubating recombinant mammalian cells expressing a human β2 adrenergic receptor, such as, for example, recombinant CHO cells, with the tested compounds for about 30 minutes at room temperature, and measuring the accumulation of cAMP, such as, for example, by homogeneous time resolved fluorescence (HTRF) technology.

In one embodiment, the at least one lignan for use of the invention is comprised in a knot-wood extract or in a knot-wood fraction as hereinabove described.

In another embodiment, the at least one lignan as disclosed herein is extracted from another plant source of lignans. Other plant sources of lignans are also disclosed and include rye, wheat, oat, barley, spelt wheat, buckwheat, millet, quinoa, amaranth, brown rice, wild rice, red rice, corncobs, sesame seeds, linseeds, peanuts, almonds, cashew nuts and walnuts.

In another embodiment of the disclosure, the at least one lignan is chemically synthesized.

The present disclosure also relates to a composition comprising a knot-wood extract as hereinabove described, or a lignan, preferably selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof and mixtures thereof.

The present disclosure also relates to a pharmaceutical composition comprising a knot-wood extract as hereinabove described, or a lignan, preferably selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof and mixtures thereof, in association with at least one pharmaceutically acceptable excipient. Examples of pharmaceutically acceptable excipients include, , water, phosphate buffer saline (PBS), PBS comprising DMSO, preferably in an amount ranging from about 1 to about 5% DMSO, anaerobic PBS, sodium bicarbonate and the like.

The present disclosure also relates to a medicament comprising a knot-wood extract as hereinabove described, or a lignan, preferably selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof and mixtures thereof.

In one embodiment, the composition, pharmaceutical composition, or medicament for use of the invention further comprises excipients, diluent and/or carriers selected with regard to the intended route of administration. Examples of excipients, diluent and/or carriers include water, phosphate buffer saline, PBS comprising DMSO, preferably in an amount ranging from about 1 to about 5% DMSO, anaerobic phosphate buffer saline, sodium bicarbonate, coloring agents, such as, for example, titane dioxide (E171), iron dioxide (E172) and brilliant black BN (E151); flavoring agents; thickeners, such as, for example, glycerol monostearate; sweeteners; coating agents, such as, for example, refined colza oil, soya oil, peanut oil, soya lecithin or fish gelatin; diluting agents, such as, for example, lactose, monohydrated lactose or starch; binding agents, such as, for example, povidone, pregelatinized starch, gums, saccharose, polyethylene glycol (PEG) 4000 or PEG 6000; disintegrating agents, such as, for example, microcrystalline cellulose or sodium carboxymethyl starch, such as, for example, sodium carboxymethyl starch type A; lubricant agents, such as, for example, magnesium stearate; flow agent, such as, for example, colloidal anhydrous silica, etc...

In one embodiment, the composition, the pharmaceutical composition or the medicament for use of the invention comprises an amount of knot-wood extract or of a lignan ranging from about 0.1 µg/mL to about 1 g/mL, preferably from about 1 µg/mL to about 100 mg/mL, more preferably from about 10 µg/mL to about 10 mg/mL. In one embodiment, the composition, the pharmaceutical composition or the medicament for use of the invention comprises an amount of knot-wood extract or of a lignan ranging from about 0.01 to about 100 mg/mL, preferably from about 0.1 to about 10 mg/mL, more preferably from about 1 to about 5 mg/mL.

The inventors herein surprisingly showed that the knot-wood extract for use of the invention, as well as the lignans for use of the invention, especially (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof have an in vitro agonist effect on the β2-adrenergic receptor (see Examples).

Consequently, the present disclosure also relates to a method for activating β2-adrenergic receptor, wherein said method comprises administering an effective amount of a knot-wood extract or fraction as disclosed herein, or of at least one lignan, preferably (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof or mixtures thereof, or of a composition, a pharmaceutical composition or a medicament as disclosed herein.

The present disclosure also relates to a method for treating a β2-adrenergic receptor related disease in a subject in need thereof, comprising administering to the subject an effective amount of a knot-wood extract or fraction as disclosed herein, or of at least one lignan, preferably (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof or mixtures thereof, or of a composition, a pharmaceutical composition or a medicament as disclosed herein.

Examples of β2-adrenergic receptor related diseases include pulmonary diseases, such as, for example, COPD (chronic obstructive pulmonary diseases), asthma, pulmonary fibrosis, chronic bronchitis and emphysema; pre-term labor; neurological disorders; cardiac disorders, such as, for example, heart failure; fibrosis; inflammation; atopic dermatitis; rheumatoid arthritis; thrombosis; allergies; ophthalmic diseases such as, for example, retinopathies or glaucoma; urological disorders such as urinary incontinence; gastrointestinal disorders such as irritable bowel syndrome, inflammatory bowel disease or spastic colitis; metabolic disorders such as obesity or diabetes.

Other examples of β2-adrenergic receptor related diseases include cancers, such as, for example, pancreatic carcinoma, prostate cancer, breast cancer or lung cancer.

According to one embodiment, the β2-adrenergic receptor related disease is a respiratory disease, preferably asthma or COPD.

The present disclosure also relates to a method of providing neuroprotection and/or cardioprotection in a subject in need thereof, comprising administering an effective amount of a knot-wood extract or fraction as disclosed herein, or of at least one lignan, preferably (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof or mixtures thereof, or of a composition, a pharmaceutical composition or a medicament as disclosed herein.

As used herein, the term "neuroprotection" refers to preventing or to treating by stopping or slowing down the development of neurologic disorders such as, for example, disorders of the central nervous system. In one embodiment, neuroprotection may aim at stopping or slowing down the loss of neurons related to these diseases. In one embodiment, said neurologic disorder is a neurodegenerative disorder, such as, for example, Parkinson disease, Huntington disease, Alzheimer disease, multiple sclerosis, Amyotrophic Lateral Sclerosis, Spinal Muscular Atrophy, spinocerebellar ataxia, multiple system atrophy, multi-infarct dementia, Alexander disease, Alpers' disease, creutzfeldt-jakob disease, Pick's disease, lysosomal storage diseases, Macrophagic Myofasciitis, Progressive supranuclear palsy or any disease wherein a progressive loss of structure or function of neurons, including death of neurons, occurs.

As used herein, the term "cardioprotection" refers to preventing or to treating by stopping or slowing down the development of cardiac disorders or cardiac events, or preventing heart damages, or preserving cardiac function. Heart damages or impairment of cardiac function may be due, for example, to drug treatments such as, for example, doxorubicin treatment; or to cardiac events, such as, for example, ischemia/reperfusion, myocardial infarction and the like.

The present disclosure also relates to a knot-wood extract or fraction as disclosed herein or to at least one lignan, preferably (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof or mixtures thereof, or to a composition, pharmaceutical composition or medicament as disclosed herein for, or for use in activating β2-adrenergic receptor

The present disclosure also relates to a knot-wood extract or fraction as disclosed herein, or to at least one lignan, preferably (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof or mixtures thereof, or to a composition, pharmaceutical composition or medicament as disclosed herein for or for use in treating a β2-adrenergic receptor related disease in a subject in need thereof. Preferably, an effective amount of the knot-wood extract or fraction as disclosed herein, or of at least one lignan, preferably (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof or mixtures thereof, or of a composition, pharmaceutical composition or medicament as disclosed herein is administered to the subject.

The present disclosure also relates to a knot-wood extract or fraction as disclosed herein, or to at least one lignan, preferably (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof or mixtures thereof, or to a composition, pharmaceutical composition or medicament as disclosed herein for or for use in providing neuroprotection and/or cardioprotection in a subject in need thereof. Preferably, an effective amount of the knot-wood extract or fraction as disclosed herein, or of at least one lignan, preferably (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof or mixtures thereof, or of a composition, pharmaceutical composition or medicament as disclosed herein is administered to the subject.

In one embodiment of the invention, the subject is an animal, preferably a mammal, more preferably a human. In one embodiment, the subject is a male. In another embodiment, the subject is a female. In another embodiment, the subject is a pet, such as, for example, a cat, a dog, a horse, a guinea pig, a hamster, a rat, a mouse, a ferret, a rabbit and the like.

In one embodiment, the subject is diagnosed with a β2-adrenergic receptor related disease, or is at risk of developing a β2-adrenergic receptor related disease. In one embodiment, said risk corresponds to a predisposition to a β2-adrenergic receptor related disease, such as, for example, a familial or genetic predisposition to a β2-adrenergic receptor related disease. In another embodiment, said risk is related to environment factors, to lifestyle factors, to the uptake of drugs or to a pre-existent infection.

According to the invention, the β2-adrenergic receptor related disease is COPD, and examples of environment factors include, without limitation, air pollution, exposure to dusts found in coal or gold mining, exposure to cadmium or isocyanates, exposure to silica dusts; while lifestyle factors include, without limitation, smoking and poor diet. According to this embodiment, the risk may be linked to the affection of the subject with an infection disease, such as, for example, HIV and tuberculosis.

In one embodiment of the disclosure, the β2-adrenergic receptor related disease is asthma, and examples of environment factors include exposure to an allergen (such as, for example, dust mites, cockroaches, animal dander, and mold), air pollution, exposure to cigarette smoke during pregnancy and after delivery, low air quality (traffic pollution or high ozone levels), exposure to indoor volatile compounds (such as, for example, formaldehyde), exposure to phthalates present in PVC, exposure to high levels of endotoxin. According to this embodiment, the risk may be linked to viral respiratory infections acquired as young children, such as, for example, respiratory syncytial virus and rhinovirus infection; atopic eczema, rhinitis, Churg-Strauss syndrome, an autoimmune disease and vasculitis, urticarial, or to obesity. Still according to this embodiment, the risk may be linked to the use of antibiotics in early life, to delivery via caesarean section. Another risk for developing asthma relates to beta-blocker medications (such as, for example, propranolol), ASA treatment, NSAID treatment, angiotensin-converting enzyme inhibitors.

In one embodiment, the subject is affected by a neurologic disorder causing loss of neurons. Examples of such neurodegenerative disorders include Parkinson disease, Huntington disease, Alzheimer disease, multiple sclerosis, Amyotrophic Lateral Sclerosis, Spinal Muscular Atrophy, spinocerebellar ataxia, multiple system atrophy, multi-infarct dementia, Alexander disease, Alpers' disease, creutzfeldt-jakob disease, Pick's disease, lysosomal storage diseases, Macrophagic Myofasciitis, Progressive supranuclear palsy or any disease wherein a progressive loss of structure or function of neurons, including death of neurons, occurs.

In one embodiment, the subject is affected with a cardiac disorder, or may have undergone heart damages or impairment of cardiac function.

In one embodiment, the knot-wood extract for use of the invention or the at least one lignan for use of the invention are used in a monotherapeutic treatment.

In another embodiment, the knot-wood extract for use of the invention or the at least one lignan for use of the invention are combined with one or more other therapeutic agents, such as, for example, muscarinic antagonist, bronchodilators, xanthine drugs, phosphodiesterase inhibitors, or an inhaled corticosteroid.

In another embodiment, the knot-wood extract for use of the invention or the at least one lignan for use of the invention are used in an add-on therapy, or is an adjuvant therapy, such as, for example, for breast cancer. As used herein, an "adjuvant therapy" refers to a treatment that is given in addition to a primary, main or initial treatment.

In an embodiment, the knot-wood extract for use of the invention, the at least one lignan for use of the invention, or the composition, pharmaceutical composition or medicament for use of the invention is administered orally. In this embodiment, oral preparations include tablets, capsules, powders, granules, and syrups. According to a first embodiment, the form adapted to oral administration is a solid form selected from the group comprising tablets, pills, capsules, soft gelatin capsules, sugarcoated pills, orodispersing/orodispersing tablets, effervescent tablets or other solids. According to a second embodiment, the form adapted to oral administration is a liquid form, such as, for example, a drinkable solution, liposomal forms and the like.

In another embodiment, the knot-wood extract for use of the invention, the at least one lignan for use of the invention, or the composition, pharmaceutical composition or medicament for use of the invention is systemically administered.

In another embodiment, the knot-wood extract for use of the invention, the at least one lignan for use of the invention, or the composition, pharmaceutical composition or medicament for use of the invention is parenterally administered, for example by intravenous injection, intramuscular injection, subcutaneous injection, intradermic injection, intraperitoneal injection, intracerebroventrocular (ICV) infusion, intracisternal injection or infusion.

According to an embodiment, the composition, pharmaceutical composition or medicament for use of the invention is in a form adapted for injection, preferably selected from the group comprising solutions, such as, for example, sterile aqueous solutions, dispersions, emulsions, suspensions, solid forms suitable for using to prepare solutions or suspensions upon the addition of a liquid prior to use, such as, for example, powder, liposomal forms and the like.

In another embodiment, the knot-wood extract for use of the invention, the at least one lignan for use of the invention, or the composition, pharmaceutical composition or medicament for use of the invention is topically administered. Examples of topical administrations include, but are not limited to, sublingual administration or dermal administration. Examples of forms adapted to topical administrations include, but are not limited to, ointment, paste, cream, gel, liposomal forms, patches, such as, for example, transdermal patches, or mucoadhesive patches (such as, for example, mucoadhesive buccal patches).

In another embodiment, the knot-wood extract for use of the invention, the at least one lignan for use of the invention, or the composition, pharmaceutical composition or medicament for use of the invention is administered by the respiratory tract, such as, for example, by inhalation spray, nasal spray, aerosol and the like. In one embodiment, the at least one lignan for use of the invention, or the composition, pharmaceutical composition or medicament for use of the invention is inhaled. Examples of devices for administration of a product by inhalation include, but are not limited to, a meter dose inhaler (MDI), a nebulizer or any other proprietary delivery device (such as, for example, Rotahaler^{®} or Autohaler^{®}).

In another embodiment, the knot-wood extract for use of the invention, the at least one lignan for use of the invention, or the composition, pharmaceutical composition or medicament for use of the invention is rectally administered. Examples of forms adapted to rectal administration include, suppositories, rectal capsules, rectal gels, rectal foams, or rectal ointments.

In an embodiment, the therapeutically effective amount of a compound may be appropriately determined in consideration of, for example, the age, weight, sex, difference in diseases, and severity of the condition of individual patients. It will be understood that the specific dose level and frequency of dosage for any particular subject or patient may be varied and will depend upon a variety of factors including the activity of the compound employed, the metabolic stability and length of action of that compound, the age, body weight, general health, sex, diet, mode and time of administration, rate of excretion, drug combination, the severity of the particular condition, and the host undergoing therapy.

In one embodiment, the therapeutically effective amount of the knot-wood extract or of the at least one lignan for use of the invention ranges from about 1 ng/kg of body weight to about 10 g/kg, preferably from about 10 ng/kg to about 1 g/kg, more preferably from about 100 ng/kg to about 100 mg/kg, and even more preferably from about 500 ng/kg to about 50 mg/kg of body weight.

In one embodiment, the daily amount of the knot-wood extract or of the at least one lignan for use of the invention administered to the subject ranges from about 0.1 mg to about 150 g/day, preferably from about 1 mg/day to about 10 g/day, more preferably from about 5 mg/day to about 5 g/day, even more preferably from about 10 mg/day to about 1 g/day, and still even more preferably is of about 50 mg/day.

### BRIEF DESCRIPTION OF THE DRAWINGS

**Figure 1** is a drawing representing a Soxhlet apparatus.
**Figure 2** is an image showing the building of a spherical harmonic based shape of a small molecule (e.g. cholesterol derivative).
**Figure 3** is a combination of images showing the SH based shape of (-)(7S,8R,8'R)-Hydroxymatairesinol, (+)-Lariciresinol, (-)-Secoisolariciresinol, and Liovil.
**Figure 4** is a graph showing the values obtained with (-)-Hydroxymatairesinol (two epimers; 7S,8R,8'R and 7R,8R,8'R; HMR), (-)(7S,8R,8'R)-Hydroxymatairesinol (HMR2), (+)-Lariciresinol (Lari), knot-wood extracts of *Picea abies* and *Abies alba* (KE2), and (-)-Secoisolariciresinol (Seco) at 10µM against β2 adrenergic receptor in an agonist assay.
**Figure 5** is a combination of graphs showing the EC50 values obtained with the knot-wood extract and with the lignans for use of the invention.
   (A): (-)-Hydroxymatairesinol (two epimers; 7S,8R,8'R and 7R,8R,8'R; HMR),
   (B): (-)(7S,8R,8'R)-Hydroxymatairesinol (HMR2),
   (C): (+)-Lariciresinol (Lari),
   (D): (-)-Secoisolariciresinol (Seco), and
   (E) knot-wood extracts of *Picea abies* and *Abies alba* (KE2) against β2 adrenergic receptor.

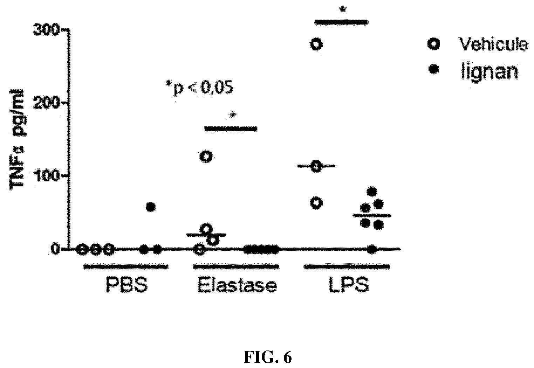

**Figure 6** is a graph showing the concentration of TNF-α in bronchoalveolar lavage fluid supernatant of mice treated with PBS, elastase or LPS.

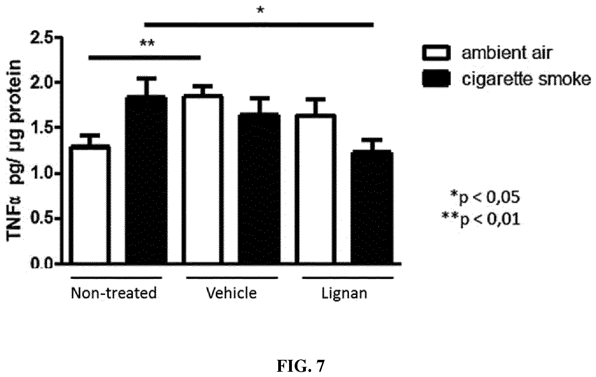

**Figure 7** is a histogram showing the concentration of TNF-α in bronchoalveolar lavage fluid supernatant in untreated mice, or in mice instilled with vehicle (PBS with 5% DMSO) or lignan for use of the invention, and exposed to ambient air or cigarette smoke.

### EXAMPLES

The present invention is further illustrated by the following examples.

### Example 1: Extraction process

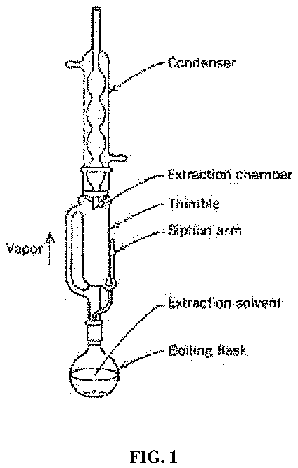

The natural crude material made of *Picea abies* and *Abies alba* knot-woods was introduced in a Soxhlet apparatus **(****Figure 1****)** in the presence of acetone as solvent. The solid sample was placed in a porous cellulose container allowing condensed solvent to saturate and pass through thereby extracting continuously active material. Condensed solvent was obtained by gently heating the solvent placed in the reservoir and by cooling subsequent solvent vapor with a condenser. The liquid condensate that dripped onto the solid sample performed the extraction which then passed through the container and went back into the reservoir by means of a siphon. The cycle was repeated continuously up to 6 hours. The knot-wood extracts were obtained after removing acetone *in vacuo.*

### Example 2: Biologic activity of the extract and lignans of the invention

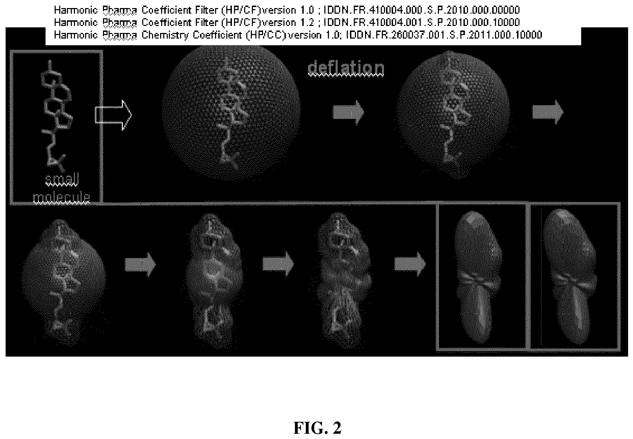

We have developed a unique spherical harmonic (SH) based molecular representation of compounds of therapeutic interest **(****Figure 2****).**

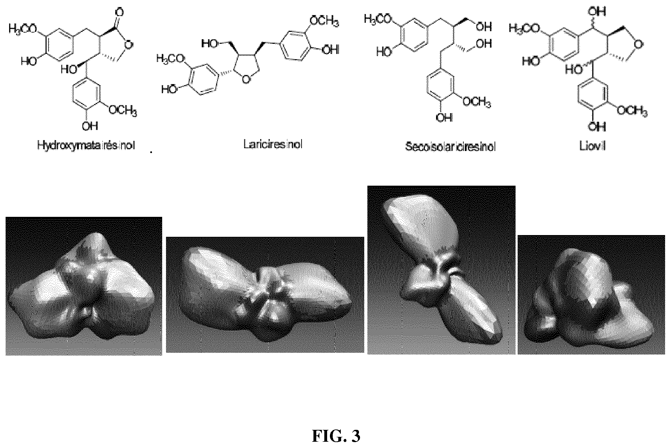

We have calculated the cognate SH shapes for (-)(7S,8R,8'R)-Hydroxymatairesinol, (+)-Lariciresinol, (-)-Secoisolariciresinol, and Liovil. **Figure 3** displays one example of the respective SH shape.

We then have tested the *in vitro* activity of (-)-hydroxymatairesinol (two epimers; 7S,8R,8'R and 7R,8R,8'R), (-)(7S,8R,8'R)-hydroxymatairesinol, (+)-lariciresinol, (-)-secoisolariciresinol, knot-wood extracts of *Picea abies* and *Abies alba* against β2-adrenergic receptor, in a cellular based assay, according to the Table 1 below (Baker J.G. (2005), Brit. J. Pharmacol., 144: 317).

**Table 1: Test of the agonist effect of compounds on the human β2-adrenergic receptor**

| **Source** | **Stimulus** | **Incubation** | **Measured component** | **Detection method** |
|---|---|---|---|---|
| Human recombinant (CHO cells) | None (0.1 µM isoproterenol for control) | 30 minutes at room temperature | cAMP | HTRF |

Mammalian recombinant cells (CHO cells) stably expressing human β2-adrenergic receptor were suspended in HBSS buffer (Invitrogen) complemented with 20 mM HEPES (pH 7.4) and 500 µM IBMX, then distributed in microplates at a density of 10⁴ cells/well and incubated for 30 min at room temperature in the presence of HBSS (basal control), the test compound or the reference agonist. For stimulated control measurement, separate assay wells contained 100 nM isoproterenol. Following incubation, the cells were lysed and the fluorescence acceptor (D2-labeled cAMP) and fluorescence donor (anti-cAMP antibody labeled with europium cryptate) were added. After 60 min at room temperature, the fluorescence transfer was measured at λex=337 nm and λem=620 and 665 nm using a microplate reader (Rubystar, BMG). The cAMP concentration was determined by dividing the signal measured at 665 nm by that measured at 620 nm (ratio). Results are expressed as a percent of the control response to 100 nM isoproterenol. The standard reference agonist is isoproterenol, which was tested in each experiment at several concentrations to generate a concentration-response curve from which its EC₅₀ value was calculated.

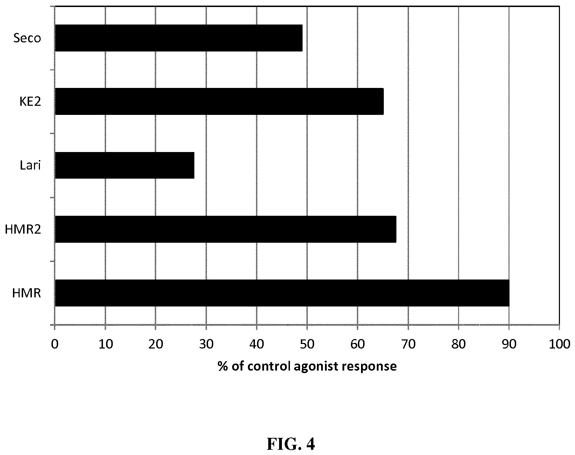

The knot-wood extract for use of the invention as well as the lignans for use of the invention were tested at a concentration of 10⁻⁵ M. Results are presented in Figure 4, and are expressed as a percentage of control agonist response, wherein 100% corresponds to the agonist response measured when cells are treated with 0.1 µM isoproterenol.

Then, we measured the corresponding EC₅₀. EC₅₀ represents the half maximal effective concentration, and refers to the concentration of a compound which induces a response halfway between the baseline and maximum.

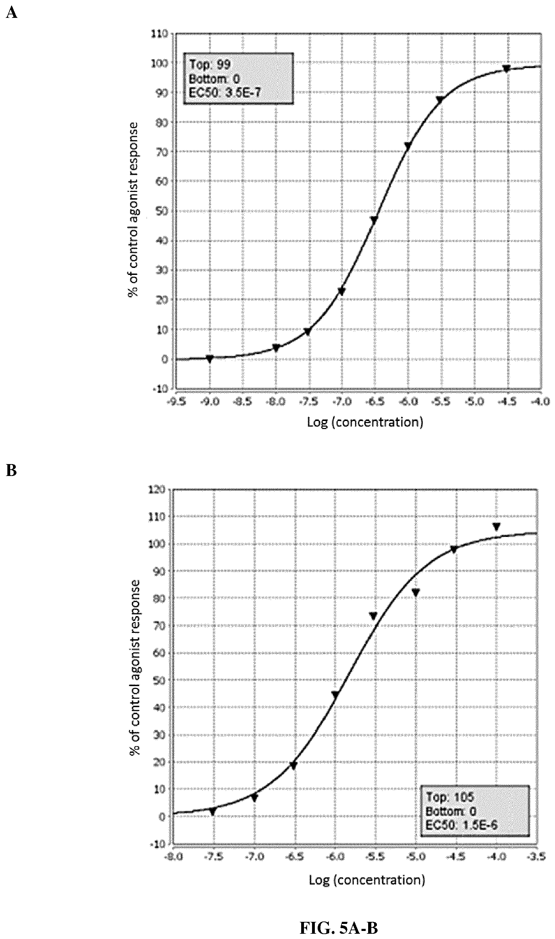

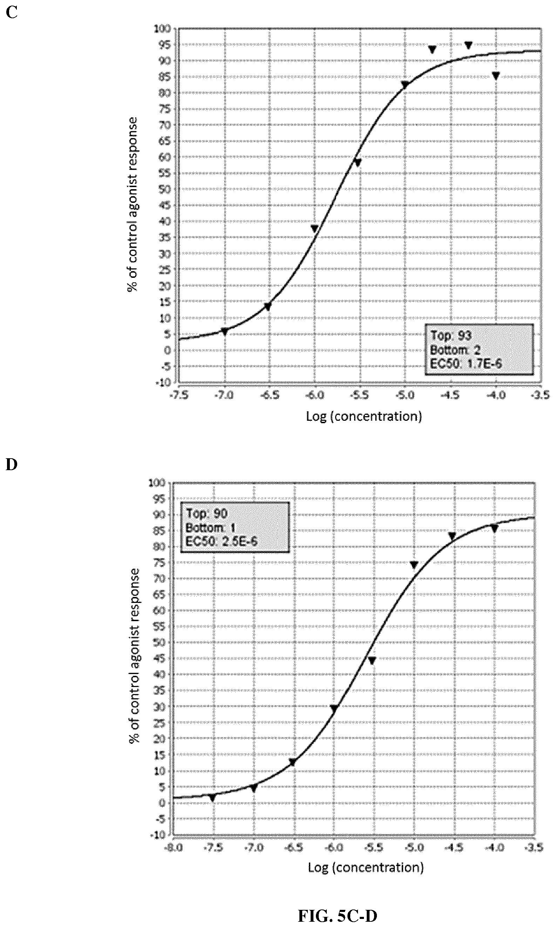

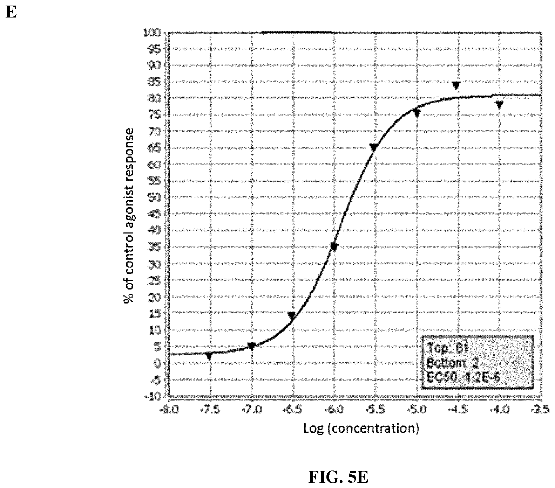

Results are presented in Table 2 below and in Figure 5.

**Table 2: EC₅₀ of the extract and of the lignans of the invention**

| **Tested compound** | **ECso (M)** |
|---|---|
| (-) hydroxymatairesinol (2 epimers: 7S,8R,8'R and 7R,8R,8'R) - HMR | 3.5.10⁻⁷ |
| (-)(7S,8R,8'R)-hydroxymatairesinol - HMR2 | 1.5.10⁻⁶ |
| (+)-lariciresinol - Lari | 1.7.10⁻⁶ |
| (-)-Secoisolariciresinol - Seco | 2.5.10⁻⁶ |
| Knot-wood extract of Picea abies and Abies alba - KE2 | 1.2.10⁻⁶ |

These results thus showed that the extract of the invention, as well as the lignans of the invention (HMR, HMR2, Seco and Lari) all exhibits a strong β2 adrenergic receptor agonist activity

### Example 3: In vivo activity of the lignans of the invention - The elastase and LPS lung injury models

The proteinase-antiproteinase imbalance hypothesis has been one of the predominant theories for explaining COPD susceptibility for nearly 50 years. The hypothesis is based on two observations: (1) a clinical study found that patients with an early-onset and familial form of emphysema were deficient for alpha1-antitrypsin, the endogenous inhibitor of neutrophil elastase; and (2) the instillation of elastase (papain) to the lungs of hamsters led to the development of emphysema. Thus, the hypothesis states that individuals who are susceptible for developing COPD are likely to have deficient antiproteolytic defences, leaving the activity of proteinases unchecked (originally this activity was largely attributed to neutrophil elastase). On the basis of these observations, two *in vivo* models had been routinely used for many years to investigate mechanisms related to COPD and for testing compounds preclinically - the elastase and the lipopolysaccharide (LPS) lung injury models (Stevenson et al, Pharmacology & Therapeutics, 2011, 130:93-105).

### Experimental protocol

Male C57B1/6 WT mice (n=35) weighing between 20 and 25 g and aged of 8 weeks were obtained from Janvier Labs (Saint Berthevin, France) and were housed in the animal facility of the laboratory, with free access to water and food, for at least 1 week before the beginning of the experimentation.

The mice were randomly divided into six groups (n = 5-6/group). At t=0, mice were submitted to intratracheal instillation with lignan of the invention (HMR with a purity of 95%, 30 mg/kg body wt dissolved in PBS containing 1% DMSO) or with PBS with 1% DMSO. At t=15 min, mice were submitted to instillation either with 30 µl of elastase (1UI diluted in PBS) or 30 µl of LPS (10 µg/ml diluted in PBS) or 30 µl of PBS containing 1% DMSO. Mice were anesthetized with 150 µl of a solution containing xylazine 10% and ketamine 10% and sacrificed after 24 hours, and the bronchoalveolar lavage (BAL) fluid was collected for subsequent analyses.

Lungs were washed twice with 1ml of PBS then centrifuged at 10,000 rpm at 4°C for 10 min, and the resulting supernatant of the BAL was aliquoted as 210 µl samples and used for TNF-α cytokine protein quantification using an ELISA kit (mouse TNF-alpha DuoSet; R&D Systems Europe, Ltd., France).

### Results

To evaluate the effects of lignan on BAL fluid, the secretion of pro-inflammatory TNF-α cytokine was measured. TNF-α is known to be a pro-inflammatory cytokine that contributes in acute pulmonary inflammation in lung injury models.

As shown in **Figure 6****,** treatment with lignan in both elastase and LPS groups significantly reduced the level of TNF-α when compared to their respective control group (p < 0.05).

This result thus strongly supports the use of the lignans of the invention for the treatment of COPD.

### Example 4: In vivo activity of the lignans of the invention - The cigarette smoke-induced lung injury model

Over the last 10 years, *in vivo* models of cigarette smoke-induced lung inflammation and lung damage have become the preferred preclinical system for investigating mechanisms related to COPD. The biggest advantage these systems offer over other models for COPD is the ability to use the primary disease-causing agent to model several key features of the disease in small animals (Stevenson et al, Pharmacology & Therapeutics, 2011, 130:93-105).

### Experimental protocol

Male C57B1/6 WT mice (n=36) weighting between 20 and 25 g and aged of 9 weeks were obtained from Janvier Labs (Saint Berthevin, France) and were housed in the animal facility of the laboratory, with free access to water and food, for at least 1 week before the beginning of the experimentation.

The mice were randomly divided into six groups (n = 6/group). Mice were submitted to intratracheal instillation with 50 µl lignan of the invention (HMR with a purity of 95%, 30 mg/kg body wt dissolved in PBS containing 5%DMSO) or with PBS with 5% DMSO daily: After 3 hours mice were exposed to ambient air (AA) or to cigarette smoke (CS; 18 cigarettes per hour) for 3 hours.

This procedure was repeated 4 days. Mice were anesthetized with 250 µl of a solution containing xylazine 10% and ketamine 10% and were sacrificed at day 5. The BAL fluid was collected for analyses.

Lungs were washed twice with 1ml of PBS then centrifuged at 10,000 rpm at 4°C for 10 min, and the resulting supernatant of the BAL was aliquoted as 210 µl samples and used for TNF-α cytokine protein quantification using an ELISA kit (mouse TNFalpha DuoSet; R&D Systems Europe, Ltd., France).

### Results

To evaluate the effects of lignan on BAL fluid, the secretion of pro-inflammatory TNF-α cytokine was measured. Indeed, TNF-α is known to be a pro-inflammatory cytokine that contributes in acute pulmonary inflammation in lung injury models.

As shown in **Figure 7****,** mice treated with lignans of the invention and exposed to cigarette smoke show a significant reduced level of TNF-α when compared to non-treated mice (p < 0.05).

This result thus strongly supports the use of the lignans of the invention for the treatment of COPD.

## Claims

1. A knot-wood extract comprising at least one lignan selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, and liovil, for use in the treatment of chronic obstructive pulmonary disease (COPD).

2. The knot-wood extract for use according to claim 1, wherein it is a dry extract.

3. The knot-wood extract for use according to claim 1 or 2, wherein the at least one lignan is at a concentration of at least 0.05 % in weight relative to the total weight of the extract.

4. The knot-wood extract for use according to claim 3, wherein the at least one lignan is at a concentration of at least 0.1 %, preferably at least 1 %, more ranging from 1 to 30 % in weight relative to the total weight of the extract.

5. The knot-wood extract for use according to any one of claims 1 to 4, wherein the extract is from at least one resinous tree, preferably from *Abies alba, Picea abies, Pinus sylvestris, Abies sibirica, Pinus sibirica, Abies balsamea, Thuja occidentalis, Northern white-cedar, Pinus cembra, Pseudotsuga menziesii, Larix decidua, Picea glauca, Picea mariana, Picea pingens, Abies pectinata, Abies lasiocarpa, Pinus banksiana, Pinus resinosa, Larix lariciana, Larix sibirica, Thuja plicata, Tsuga hetewphylla, Pinus contorta, and Juniperus virginiana.*

6. The knot-wood extract for use according to claim 5, wherein the extract is from *Abies alba* and/or *Picea abies.*

7. A lignan selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, and liovil, for use in the treatment of chronic obstructive pulmonary disease (COPD).

8. A pharmaceutical composition comprising a knot-wood extract as defined in any one of claims 1 to 6 or a lignan as defined in claim 7, and at least one pharmaceutically acceptable excipient, for use in the treatment of chronic obstructive pulmonary disease (COPD).

9. The pharmaceutical composition for use according to claim 8, wherein it comprises an amount of said knot-wood extract or of said lignan ranging from 0.1 µg/mL to 1 g/mL, preferably from 1 µg/mL to 100 mg/mL, more preferably from 10 µg/mL to 10 mg/mL.

10. The pharmaceutical composition for use according to claim 8, wherein it comprises an amount of said knot-wood extract or of said lignan ranging from 0.01 to 100 mg/mL, preferably from 0.1 to 10 mg/mL, more preferably from 1 to 5 mg/mL.

11. The pharmaceutical composition for use according to any one of claims 8 to 10, wherein it is intended for oral, systemic, parenteral, topical, respiratory tract or rectal administration.

12. The pharmaceutical composition for use according to any one of claims 8 to 11, wherein it comprises a therapeutically effective amount of said knot-wood extract or of said lignan ranging from 1 ng/kg to 10 g/kg of body weight, preferably from 10 ng/kg to 1 g/kg of body weight, more preferably from 100 ng/kg to 100 mg/kg of body weight, and even more preferably, from 500 ng/kg to 50 mg/kg of body weight.

13. The pharmaceutical composition for use according to any one of claims 8 to 12, wherein the daily amount of said knot-wood extract or of said lignan in the subject ranges from 0.1 mg/day to 150 g/day, preferably from 1 mg/day to 10 g/day, more preferably from 5 mg/day to 5 g/day, even more preferably from 10 mg/day to 1 g/day, and still even more preferably 50 mg/day.

## Patentansprüche

1. Knotenholzextrakt, umfassend mindestens ein Lignan, ausgewählt aus der Liste umfassend (-) Hydroxymatairesinol (zwei Epimeren; 7S,8R,8'R und 7R,8R,8'R), (-)(7S, 8R, 8'R)-Hydroxymatairesino1, (+)-Lariciresino1, (-)-Secoisolariciresino1 und Liovil zur Behandlung der chronisch obstruktiven Lungenerkrankung (COPD).

2. Knotenholzextrakt zur Verwendung nach Anspruch 1, **dadurch gekennzeichnet, dass** es sich um einen Trockenextrakt handelt.

3. Knotenholzextrakt zur Verwendung nach Anspruch 1 oder 2, wobei das mindestens eine Lignan in einer Konzentration von mindestens 0,05 Gew.-%, bezogen auf das Gesamtgewicht des Extrakts, vorliegt

4. Knotenholzextrakt zur Verwendung nach Anspruch 3, wobei das mindestens eine Lignan in einer Konzentration von mindestens 0,1 Gew.-%, vorzugsweise mindestens 1 Gew.-%, mehr im Bereich von 1 bis 30 Gew.-%, bezogen auf das Gesamtgewicht des Extrakts vorliegt.

5. Knotenholzextrakt zur Verwendung nach einem der Ansprüche 1 bis 4, wobei der Extrakt aus mindestens einem harzführenden Baum stammt, vorzugsweise aus *Abies alba, Picea abies, Pinus sylvestris, Abies sibirica, Pinus sibirica, Abies balsamea, Thuja occidentalis, Northern white-cedar, Pinus cembra, Pseudotsuga menziesii, Larix decidua, Picea glauca, Picea mariana, Picea pingens, Abies pectinata, Abies lasiocarpa, Pinus banksiana, Pinus resinosa, Larix lariciana, Larix sibirica, Thuja plicata, Tsuga hetewphylla, Pinus contorta, and Juniperus virginiana.*

6. Knotenholzextrakt zur Verwendung nach Anspruch 5, wobei der Extrakt aus *Abies alba* und/oder *Picea abies* stammt.

7. Lignan ausgewählt aus der Liste umfassend (-)-Hydroxymatairesinol (zwei Epimeren; 7S,8R,8'R und 7R,8R,8'R), (-)(7S,8R,8'R)-Hydroxymatairesinol, (+)-Lariciresinol, (-)-Secoisolariciresinol und Liovil zur Anwendung zur Behandlung der chronisch obstruktiven Lungenerkrankung (COPD).

8. Pharmazeutische Zusammensetzung, umfassend einen Knotenholzextrakt nach einem der Ansprüche 1 bis 6 oder ein Lignan nach Anspruch 7 und mindestens einen pharmazeutisch verträglichen Hilfsstoff zur Verwendung bei der Behandlung von chronisch obstruktiver Lungenerkrankung (COPD).).

9. Pharmazeutische Zusammensetzung zur Verwendung nach Anspruch 8, wobei sie eine Menge dieses Knotenholzextrakts oder dieses Lignans im Bereich von 0,1 µg/ml bis 1 g/ml, vorzugsweise von 1 µg/ml bis 100 mg/ml, stärker bevorzugt von 10 µg/ml bis 10 mg/ml umfasst.

10. Pharmazeutische Zusammensetzung zur Verwendung nach Anspruch 8, wobei sie eine Menge dieses Knotenholzextrakts oder dieses Lignans im Bereich von 0,01 bis 100 mg/ml, vorzugsweise von 0,1 bis 10 mg/ml, stärker bevorzugt von 1 bis 5 mg/ml umfasst.

11. Pharmazeutische Zusammensetzung zur Verwendung nach einem der Ansprüche 8 bis 10, wobei sie zur oralen, systemischen, parenteralen, topischen, respiratorischen oder rektalen Verabreichung bestimmt ist.

12. Pharmazeutische Zusammensetzung zur Verwendung nach einem der Ansprüche 8 bis 11, wobei sie eine therapeutisch wirksame Menge dieses Knotenholzextrakts oder dieses Lignans im Bereich von vorzugsweise 1 ng/kg bis 10 g/kg Körpergewicht, von 10 ng/kg bis 1 g/kg Körpergewicht, stärker bevorzugt von 100 ng/kg bis 100 mg/kg Körpergewicht und noch stärker bevorzugt von 500 ng/kg bis 50 mg/kg Körpergewicht umfasst.

13. Pharmazeutische Zusammensetzung zur Verwendung nach einem der Ansprüche 8 bis 12, wobei die tägliche Menge dieses Knotenholzextrakts oder dieses Lignans in dem Patienten im Bereich von 0,1 mg/Tag bis 150 g/Tag, vorzugsweise von 1 mg, liegt /Tag bis 10 g/Tag, stärker bevorzugt 5 mg/Tag bis 5 g/Tag, noch stärker bevorzugt 10 mg/Tag bis 1 g/Tag und noch stärker bevorzugt 50 mg/Tag reicht.

## Revendications

1. Extrait de bois noueux comprenant au moins un lignane choisi dans la liste comprenant le (-)-hydroxymatairésinol (deux épimères : 7S,8R,8'R et 7R,8R,8'R), le (-)(7S,8R,8'R)-hydroxymatairésinol, le (+)-laricirésinol, le (-)-sécoisolaricirésinol, et le liovil, destiné à être utilisé dans le traitement de la broncho-pneumopathie chronique obstructive (BPCO).

2. Extrait de bois noueux destiné à être utilisé selon la revendication 1, qui est un extrait sec.

3. Extrait de bois noueux destiné à être utilisé selon la revendication 1 ou 2, dans lequel le au moins un lignane est à une concentration d'au moins 0,05 % en poids par rapport au poids total de l'extrait.

4. Extrait de bois noueux destiné à être utilisé selon la revendication 3, dans lequel le au moins un lignane est à une concentration d'au moins 0,1 %, de préférence d'au moins 1 %, mieux encore de 1 à 30 % en poids par rapport au poids total de l'extrait.

5. Extrait de bois noueux destiné à être utilisé selon l'une quelconque des revendications 1 à 4, dans lequel l'extrait provient d'au moins un arbre résineux, de préférence *Abies alba, Picea abies, Pinus sylvestris, Abies sibirica, Pinus sibirica, Abies balsamea, Thuja occidentalis,* le cèdre blanc du Canada, *Pinus cembra, Pseudotsuga menziesii, Larix decidua, Picea glauca, Picea mariana, Picea pingens, Abies pectinata, Abies lasiocarpa, Pinus banksiana, Pinus resinosa, Larix lariciana, Larix sibirica, Thuja plicata, Tsuga hetewphylla, Pinus contorta,* et *Juniperus virginiana.*

6. Extrait de bois noueux destiné à être utilisé selon la revendication 5, dans lequel l'extrait provient *d'Abies alba* et/ou *Picea abies.*

7. Lignane choisi dans la liste comprenant le (-)-hydroxymatairésinol (deux épimères : 7S,8R,8'R et 7R,8R,8'R), le (-)(7S,8R,8'R)-hydroxymatairésinol, le (+)-laricirésinol, le (-)-sécoisolaricirésinol, et le liovil, destiné à être utilisé dans le traitement de la broncho-pneumopathie chronique obstructive (BPCO).

8. Composition pharmaceutique comprenant un extrait de bois noueux tel que défini dans l'une quelconque des revendications 1 à 6 ou un lignane tel que défini dans la revendication 7, et au moins un excipient pharmaceutiquement acceptable, destinés à être utilisés dans le traitement de la broncho-pneumopathie chronique obstructive (BPCO).

9. Composition pharmaceutique destinée à être utilisée selon la revendication 8, qui comprend une quantité dudit extrait de bois noueux ou dudit lignane située dans la plage allant de 0,1 µg/ml à 1 g/ml, de préférence de 1 µg/ml à 100 mg/ml, mieux encore de 10 µg/ml à 10 mg/ml.

10. Composition pharmaceutique destinée à être utilisée selon la revendication 8, qui comprend une quantité dudit extrait de bois noueux ou dudit lignane située dans la plage allant de 0,01 à 100 mg/ml, de préférence de 0,1 à 10 mg/ml, mieux encore de 1 à 5 mg/ml.

11. Composition pharmaceutique destinée à être utilisée selon l'une quelconque des revendications 8 à 10, qui est destinée à être administrée par voie orale, systémique, parentérale, topique, rectale, ou par les voies respiratoires.

12. Composition pharmaceutique destinée à être utilisée selon l'une quelconque des revendications 8 à 11, qui comprend une quantité efficace du point de vue thérapeutique dudit extrait de bois noueux ou dudit lignane située dans la plage allant de 1 ng/kg à 10 g/kg de poids corporel, de préférence de 10 ng/kg à 1 g/kg de poids corporel, mieux encore de 100 ng/kg à 100 mg/kg de poids corporel, et plus particulièrement de 500 ng/kg à 50 mg/kg de poids corporel.

13. Composition pharmaceutique destinée à être utilisée selon l'une quelconque des revendications 8 à 12, dans laquelle la quantité journalière dudit extrait de bois noueux ou dudit lignane dans le sujet est située dans la plage allant de 0,1 mg/jour à 150 g/jour, de préférence de 1 mg/jour à 10 g/jour, mieux encore de 5 mg/jour à 5 g/jour, plus particulièrement de 10 mg/jour à 1 g/jour, et tout spécialement de 50 mg/jour.
